# NEW EUROPEAN PATENT SPECIFICATION

(11) **EP 0 307 154 B2**
(45) Date of publication and mention of the opposition decision: **04.12.1996**
(45) Mention of the grant of the patent: 04.08.1993
(21) Application number: 88308197.8
(22) Date of filing: 05.09.1988
(51) Int. Cl.: C12P 7/62, C12P 7/64

(54) **Preparation of diglycerides**
Herstellung von Diglyceriden
Préparation de diglycérides

(30) Priority: 09.09.1987 JP 225866/87
(43) Date of publication of application: 15.03.1989
(73) Proprietor: Kao Corporation, Chuo-ku Tokyo (JP)
(72) Inventor: Hirota, Yoshitaka, Kashima-gun Ibaraki (JP); Kohori, Jun, Kashima-gun Ibaraki (JP); Kawakara, Yoshihaur, Sawara-shi Chiba (JP)
(74) Representative: Bannerman, David Gardner

(56) References cited:
- EP-A- 0 188 725
- PATENT ABSTRACTS OF JAPAN, vol. 11, no. 202 (C-432)[2649], 30th June 1987; & JP-A-62 25 987 (MEITO SANGYO K.K.)
- PATENT ABSTRACTS OF JAPAN, vol. 11, no. 115 (C-415)[2562], 10th April 1987; & JP-A-61 257 192 (AGENCY OF INT. SCIENCE & TECHNOL.)
- CHEMICAL ABSTRACTS, vol. 109, no. 19, 7th November 1988, page 598, abstract no. 168998z, Columbus, Ohio, US; & JP-A-63 133 992 (KAO CORP.) 06-06-1988
- CHEMICAL ABSTRACTS, vol. 107, no. 11, 14th September 1987, page 573, abstract no. 95296r, Columbus, Ohio, US; & JP-A-62 19 090 (MEITO CO., LTD) 27-01-1987
- PATENT ABSTRACTS OF JAPAN, vol. 6, no. 68 (C-100)[946], 30th April 1982; & JP-A-57 8787 (FUJI SEIYU K.K.)
- Biotech. Letters, 1 (1979), pp.211-216
- JAOCS, 61(2) 1984, pp. 191-195
- JAOCS, 65(6) 1988, pp. 927-931
- Biochim. Biophys. Acta, 489, (1977), pp. 415-422

## Description

This invention relates to a method for preparation of diglycerides using lipase, more particularly to a method for preparation of high purity diglycerides by the reaction between fatty acids or esters of fatty acids and glycerol using immobilized lipase or endo-lipase which occurs preferentially at 1st and 3rd positions of the glycerides, here referred to as 1,3-position selective immobilized lipase or endo-lipase.

Diglycerides have been used as a raw material in the cosmetic and medical fields and as plastically-improving additives for fats in the food industry.

Diglycerides have usually been prepared by esterification of glycerol with fatty acids and by alcohol exchange reactions between glycerol and fats. For example, in the alcohol exchange reaction (glycerolysis) the reaction is carried at a temperature of 200 to 240°C for 2 to 6 hr with about 0.1% of a calcium hydroxide catalyst.

The resulting reaction product contains monoglycerides and triglycerides in addition to diglycerides. That is, in the prior art the composition is determined by random distribution and a high proportion of monoglyceride is formed under conditions that minimize triglyceride production and on the contrary a high proportion of triglyceride is formed under conditions that minimize monoglyceride production. It is therefore difficult to obtain diglyceride preferentially and at a high yield.

On the other hand, a method for preparation of glycerides using lipase, which is a fat hydrolysis enzyme, has been proposed. A high quality glyceride is obtained because the product is not subjected to high temperature treatment. For example, Tsujisaka et al. synthesized glyceride from a fatty acid and glycerol using an aqueous lipase solution of Rhizopus delemar and obtained a composition of 34% monoglyceride, 36% diglyceride and 28% triglyceride with 70% consumption of the fatty acid. See Japanese patent publication 51-7754 (1976).

However, in this method the required addition of an aqueous lipase solution and protein solution for the reaction adversely affect the yield of ester due to the water present in the system. The ester synthesis yield is defined as the ratio of fatty acid consumed by the esterification to the fatty acid originally added. In the case of low yield, fatty acid as well as hydroxyl groups of the glycerol remain unreacted; consequently the proportion of glycerol and monoglyceride is high. As a result, removal of the fatty acid and monoglyceride requires a large scale facility and the ratio of diglyceride to raw material is low. This method is thus difficult to use in practice.

Yamane et al used lipase of Chromobacterium viscosum var paralipolyticum in a system with 3 to 4% of water to react oleic acid with glycerol to synthesize glyceride and obtained a glyceride composition comprising approximately 36% monoolein , 34% diolein, and 10% triolein with approximately an 80% fatty acid consumption. (JAOCS., 61(4), 776(1984)). However, this method is also impractical because of the high yield of monoglyceride.

A method to obtain a high concentration of diglyceride has been proposed by Kakuta at al. (Japanese Patent Provisional Publication No. 63(1988)-25987). According to this method, a reaction is carried out essentially without any addition of water using the micro-organism Alcalilipase to increase the yield. According to the description the esterification between stearic acid and glycerol using lipase obtained from Alcali genus, which yielded the highest diglyceride concentration, gives a glyceride composition of 25% monostearin, 70% distearin, 3% tristearin, and 2% stearic acid at an ester yield of 97%. Although the method resulted in a good ester to synthesis yield, because not only a considerable amount of mono glyceride was contained in the product but also a special commercially-unavailable lipase (i.e. Alcalilipase) was required, this method is impractical for industrial application to obtain high purity diglyceride at a high yield.

We have now found that immobilized 1,3 position selective lipase and 1,3 position selective endo-lipase have excellent esterification properties for the esterification of fatty acids or lower alcohol esters thereof with glycerol and that high purity diglyceride can be obtained at a high yield by removing the water or lower alcohol generated by the reaction from the system.

According to the present invention we therefore provide a method for preparing a diglyceride from a saturated or unsaturated fatty acid, having 4 to 22 carbon atoms, or a lower alcohol ester thereof, and glycerol, which comprises the step of reacting the fatty acid or a lower alcohol ester thereof with glycerol in the presence of an immobilized lipase or an endo-lipase that reacts with glycerides preferentially at their 1st and 3rd positions, while water or lower alcohol produced by the reaction is removed. Preferably, at least 1.5 mol of the fatty acids or lower alcohol ester is used per mole of glycerol, and more preferably 1.5 to 3.5 moles of fatty acid or ester.

The invention will be described in detail hereinafter. The 1,3 position selective immobilized lipase or endo-lipase used in the present invention is obtained by a known method for immobilizing 1,3 selective lipase. Known methods for immobilization are described in, for example, "Immobilized enzymes" edited by Ichiro Senbatake, Kodansha press, on page 9 to 85 and "Immobilized biocatalysts" edited by Ichiro Senbatake, Kodansha press, on pages 12 to 101. Among many methods, a method in which lipase is immobilised on an ion exchange resin is preferably used. Examples of 1,3 position selective lipase include microorganism-derived lipase such as Rhizopus genus, Aspergillus genus, and Mucor genus and pancreas lipase. For example, lipase from Rhizopus delemar, Rhizopus japonicus, Rhiopus niveus, Aspergillus niger, Mucor javanicus, and Mucor miehei may be used. An example of commercially available 1,3 position selective immobilized lipase includes that available as "Lipozyme 3A", from NOVO INDUSTRY AS. An example of 1,3 position selective endo-lipase (a bacteria to which 1,3 position selective lipase is adsorbed or bonded) is "OLIPASE" commercially available from OSAKA SAIKIN KENKYUSHO. These immobilized lipases or endo-lipases maintain their performance under reduced pressure conditions, hence a water retention capability is required. For this purpose a lipase immobilized on an ion-exchange resin is preferably used.

Saturated or unsaturated fatty acids with 4 to 22 carbon atoms are used in the present invention. Examples include butyric acid, valeric acid, capric acid, enanthic acid, caprylic acid, pelargonic acid, capric acid, undecaroic acid, lauric acid, myristic acid, palmitic acid, zoomaric acid, stearic acid, oleic acid, elaidic acid, linoleic acid, linolenic acid, arrachidonic acid, gadoleic acid, arrachidic acid, behenic acid, and erucic acid. Esters of the above mentioned fatty acids and lower alcohols with 1 to 3 carbon atoms may be used instead of the corresponding fatty acid. Lower alcohols with 1 to 3 carbon atoms include methanol, ethanol, propanol, and isopropanol. These fatty acids or esters of fatty acids may be used in combination or alone.

Examples of the method for removing water or lower alcohol generated in the reaction from the system to increase the ester synthesis yield include dehydration or alcohol removal by pressure reduction, bubbling of a dried inert gas into the reaction vessel, and use of adsorbents such as molecular sieves. Dehydration or alcohol removal under reduced pressure (lower than atmospheric pressure) is preferably used because of less contamination of the reaction system. Details of the method is described for example, in Japanese Patent Provisional Publication No. 57(1982)-8787.

At least 1.5 mol, preferably 1.5 to 3.5 mol, more preferably 1.5 to 2.2 mol, per 1 mol of glycerol, of a fatty acid or a lower alcohol ester thereof and the above-mentioned lipase preparation which contains 200 to 10,000 units of the lipase per 1g of the fatty acid or a lower alcohol ester thereof, are mixed with each other. The mixture is reacted at a temperature of 20 to 100°C, preferably 40 to 70°C.

If the ratio of the fatty acid to glycerol is less than 1.5 mol/mol, the monoglyceride content of the product would be too high and the yield of diglyceride would be not satisfactory.

Water or lower alcohol generated during the reaction is removed by one of the above-mentioned methods. The reaction time ranges from 2 to 72 hours, but a reaction time of 2 to 24 hours is preferred because prolonged reaction causes an increase in triglyceride yield and a decrease in diglyceride purity.

Water in the reaction system resulting from the lipase preparation is removed and the reaction is carried out under essentially water-free conditions. Solvents such as hexane, octane, and petroleum ether may be used but this is undesirable as they need to be removed and the product purified. The water content in the lipase preparation preferably ranges from 0.1 to 20% by weight, and more preferably from 2 to 6% by weight.

The lipase preparation is separated from the reaction products, and methods such as molecular distillation and thin film distillation alone or in combination may be employed depending on the case. This process inhibits migration of acyl groups, and provides re-use of unreaction raw materials. Purified diglyceride is obtained at a high yield and high purity. The lipase recovered can be re-used.

As mentioned heretobefore by the esterification of fatty acids or lower alcohol esters of fatty acids with glycerol using 1,3 position selective lipase in accordance with the present invention, diglycerides can be obtained at a high purity and high yield, which can not be achieved by conventional methods. The invention will be described in detail referring to the following non-limiting examples:

### EXAMPLE 1

20.0g of a commercially available lipase preparation which is 1,3 position selective immobilized lipase (brand name; "Lipozyme 3A" from NOVO INDUSTRY AS, lipase from Mucor miehei immobilized on macro-porous anion exchange resin), 86.0 (0.305 mol) of oleic acid, and 14.0g (0.152 mol) of glycerol were mixed and reacted with stirring at 40°C for 10 hours. During esterification the reaction system was maintained at a reduced pressure of 150 mmHg to remove water generated by the reaction and to increase the ester synthesis yield. After the reaction the residual amount of fatty acid was determined by alkali titration. After separation of the lipase preparation from the reaction product, a sample of the reaction product was subjected to trimethylsilylation and analyzed by gas chromatography to determine the proportions of triglyceride, diglyceride, and monoglyceride. The result obtained is shown in Table 1. This shows that the diglyceride obtained contained 95% of 1,3-diolein and 5% of 1,2-diolein.

### EXAMPLE 2

10.0g of a commercially available lipase preparation which is 1,3 position selective endolipase (brand name; "Olipase" from OSAKA SAIKIN KENKYUSHO, 22800 units/g, endo-lipase from Rhizopus japonicus), 86.0g (0.305 mol) of oleic acid, and 14.0g (0.152 mol) of glycerol were mixed, and reacted with stirring at 40°C for 21 hours. The pressure in the system was maintained at 320 mmHg to remove water from the system. After the reaction the ester synthesis yield and glyceride composition were determined by the same procedures as used in EXAMPLE 1. The result is shown in Table 1.

### REFERENCE EXAMPLE 1

5.6g of commercially available lipase preparation which is neither immobilized nor endolipase (brand name; "Talipase" 10,000 units/g, provided from TANABE SEIYAKU Co. Ltd., originated form Rhizopus delemar), 86.0g of oleic acid, and 14.0g of glycerin were mixed, and reacted under the same conditions and analyzed by the same procedure as in EXAMPLE 1 . The result is shown in Table 1.

### REFERENCE EXAMPLE 2

6.4g of a commercially available lipase preparation which acts non-selecting on glycerol (brand name; "Lipase OF" 360,000 units/g, from THE MEITO SANGYO Co. Ltd., from Candida genus), 86.0g of olein, and 14.og of glycerol were mixed, and the mixture was reacted under the same conditions and analyzed to determine the ester synthesis yield and glyceride composition. The result is shown in Table 1. 69% of 1,3-diolein and 31% of 1,2-diolein are contained in the diglyceride.

### EXAMPLE 3

25g of a commercially available lipase preparation (the same lipase as used in EXAMPLE 1), 130g (0.439 mol) of methyl oleate, and 20g (0.217 mol) of glycerol were mixed, and reacted with stirring at 40°C for 7 hours. The pressure was reduced to 150 mmHg to remove methanol generated during the reaction from the system. After the reaction the lipase preparation was filtered off and 5.2g of methyl oleate was separated and removed by distillation. The glyceride composition was determined by the same procedure as used in EXAMPLE 1 and a result of 12.0% monoolein. 81.1% of diolein, and 6.9% of triolein was obtained.

### EXAMPLE 4

20.0 commercially available lipase preparation (the same lipase as used in EXAMPLE 1), 100g of (0.354 mol) fatty acid obtained by decomposition of rapeseed oil, and 16.3g (0.177 mol) of glycerol were mixed, and reacted at 60°C for 6 hours at a reduced pressure of 5 mmHg. After the reaction the ester synthesis yield and glyceride composition were determined by the same procedures as used in EXAMPLE 1. The result is shown in Table 2.

### REFERENCE EXAMPLE 3

100g of rapeseed oil, 10g of glycerol, 1g of activated carbon, and 0.01g of calcium hydroxide were mixed, and reacted at 235°C for 3 hours in a stream of nitrogen. After the reaction, the residual calcium hydroxide was neutralized with phosphoric acid, and filtered off from the reaction product together with activated carbon. Then a sample of the reaction product was subjected to trimethylsilylation and its glyceride composition was determined using gas chromatography in the same manner as in EXAMPLE 1. The result is shown in Table 2.

### REFERENCE EXAMPLE 4

100g of rapeseed oil, 30g of glycerol 1.3g of activated carbon, and 0.01g of calcium hydroxide were mixed, and reacted in the same manner as in REFERENCE EXAMPLE 3, and the glyceride composition of the reaction product was determined. The result is shown in Table 2. 69.6% of 1,3-diglyceride, 30.4% of 1,2-diglyceride are contained in the diglyceride.

### EXAMPLE 5

100g of fatty acid obtained by decomposition of corn oil, 16.5g of glycerol, and 20.0% of a commercially available lipase preparation (the same lipase as used in EXAMPLE 1) were mixed, and reacted with stirring at 40°C for 8 hours under a reduced pressure of 40 mmHg. After the reaction the ester synthesis yield and glyceride composition were determined by the same procedures as in EXAMPLE 1. The result is shown in Table 2.

**Table 1**

| | Ester synthesis yield (%) | Monoglyceride | Diglyceride | Triglyceride |
|---|---|---|---|---|
| Example 1 | 95.3 | 13.5 % | 80.1 % | 6.4 % |
| Example 2 | 96.0 | 11.6 | 79.4 | 9.0 |
| Example 3 | - | 12.0 | 81.1 | 6.9 |
| Reference example 1 | 48.2 | 49.5 | 50.3 | 0.2 |
| Reference example 2 | 93.3 | 15.2 | 50.8 | 34.0 |

**Table 2**

| | Ester synthesis yield (%) | Monoglyceride | Diglyceride | Triglyceride |
|---|---|---|---|---|
| Example 4 | 94.6 | 15.6 | 79.3 | 5.1 |
| Reference example 3 | - | 27.1 | 48.8 | 24.1 |
| Reference example 4 | - | 55.5 | 42.4 | 2.1 |
| Example 5 | 95.7 | 15.5 | 78.9 | 5.6 |

## Claims

1. A method for preparing a diglyceride from a saturated or unsaturated fatty acid having 4 to 22 carbon atoms, or a lower alcohol ester thereof, and glycerol, which comprises the step of reacting at least 1.5 moles of the fatty acid or lower alcohol ester thereof with one mole of glycerol in the presence of an immobilized lipase or an endo-lipase that reacts with glycerides preferentially at their 1st and 3rd positions, while water or lower alcohol produced by the reaction is removed under reduced pressure.

2. A method as claimed in claim 1, in which 1.5 to 3.5 moles of the fatty acid or lower alcohol ester thereof is used per mole of glycerol.

3. A method as claimed in claim 1, in which the lipase has been immobilised on an ion-exchange resin.

4. A method as claimed in claim 1, in which 200 to 10,000 units of the lipase is used per gram of the fatty acid or lower alcohol ester thereof.

5. A method as claimed in claim 1, in which the reaction is carried out in a non-acqueous medium and the lipase preparation contains 0.1 to 20 percent by weight of water.

## Patentansprüche

1. Verfahren zur Herstellung eines Diglycerids aus einer gesättigten oder ungesättigten Fettsäure mit 4 bis 22 Kohlenstoffatomen oder einem niederen Alkoholester davon und Glycerin, umfassend den Schritt der Umsetzung von wenigstens 1,5 Mol der Fettsäure oder deren niederem Alkoholester, mit einem Mol Glycerin in Gegenwart einer immobilisierten Lipase oder Endo-Lipase (das heißt eine 1,3-Stellung selektive Lipase, adsorbiert auf oder an ein Bakterium gebunden), die mit Glyceriden bevorzugt an deren ersten und dritten Stellungen reagiert, während durch die Reaktion erzeugtes Wasser oder niederer Alkohol unter vermindertem Druck entfernt wird.

2. Verfahren nach Anspruch 1, bei dem 1,5 bis 3,5 Mol Fettsäure oder deren niedrigem Alkoholester pro Mol Glycerin verwendet werden.

3. Verfahren nach Anspruch 1, bei dem die Lipase auf einem Ionen-austauschenden Harz immobilisiert wurde.

4. Verfahren nach Anspruch 1, bei dem 200 bis 10.000 Einheiten Lipase pro Gramm der Fettsäure oder deren niedrigem Alkoholester verwendet werden.

5. Verfahren nach Anspruch 1, bei dem die Reaktion in einem nichtwässrigen Medium durchgeführt wird und die Lipasepräparation 0,1 bis 20 Gew.-% Wasser enthält.

## Revendications

1. Procédé pour préparer un diglycéride à partir d'un acide gras, saturé ou non, ayant de 4 à 22 atomes de carbone, ou d'un ester d'alcool inférieur de celui-ci, et de glycérol, qui comprend l'étape consistant à faire réagir au moins 1,5 mole de l'acide gras, ou d'un ester d'alcool inférieur de celui-ci, avec une mole de glycérol en présence d'une lipase immobilisée ou d'une endolipase (c'est-à-dire une lipase sélective des positions 1 et 3 adsorbée sur ou liée à une bactérie) qui réagit avec les glycérides, de préférence, en leurs première et troisième positions, en éliminant l'eau ou l'alcool inférieur produit par la réaction sous pression réduite.

2. Procédé tel que revendiqué dans la revendication 1, dans lequel on utilise de 1,5 à 3,5 mole de l'acide gras, ou de l'ester d'alcool inférieur de celui-ci, par mole de glycérol.

3. Procédé tel que revendiqué dans la revendication 1, dans lequel on a immobilisé la lipase sur une résine échangeuse d'ions.

4. Procédé tel que revendiqué dans la revendication 1, dans lequel on utilise de 200 à 10000 unités de la lipase par gramme d'acide gras ou d'ester d'alcool inférieur de celui-ci.

5. Procédé tel que revendiqué dans la revendication 1, dans lequel on effectue la réaction dans un milieu non aqueux et la préparation de lipase contient de 0,1 à 20 pour-cent en poids d'eau.
